# FASCICULE DE BREVET EUROPEEN

(11) **EP 0 660 698 B1**
(45) Date de publication et mention de la délivrance du brevet: **06.08.1997**
(21) Numéro de dépôt: 93920895.5
(22) Date de dépôt: 17.09.1993
(51) Int. Cl.: A61K 7/06

(54) **COMPOSITION COSMETIQUE OU DERMATOLOGIQUE CONTENANT AU MOINS UNE SAPONINE DE TYPE GINSENOSIDE, ET SES APPLICATIONS, NOTAMMENT POUR LE SOIN DES CHEVEUX**
KOSMETISCHE ODER DERMATOLOGISCHE ZUSAMMENSETZUNG, DIE MINDESTENS EIN SAPONIN VOMGINSENOSID-TYP ENTHÄLT, ZUR HAARPFLEGE
COSMETIC OR DERMATOLOGIC COMPOSITION CONTAINING AT LEAST ONE SAPONINE OF THE GINSENOSIDE TYPE, AND ITS APPLICATIONS PARTICULARLY TO HAIR CARE

(30) Priorité: 17.09.1992 FR 9211104
(43) Date de publication de la demande: 05.07.1995
(73) Titulaire: LVMH RECHERCHE, 92752 Nanterre (FR)
(72) Inventeur: MEYBECK, Alain Les Poissons, F-92400 Courbevoie (FR); BONTE, Frédéric, F-92400 Courbevoie (FR); DUMAS, Marc, F-92700 Colombes (FR)
(74) Mandataire: Portal, Gérard
(86) Numéro de dépôt international: FR9300899
(87) Numéro de publication internationale: WO9406402

(56) Documents cités:
- WO-A-92/16186
- CHEMICAL ABSTRACTS, vol. 103, no. 3, 22 Juillet 1985, Columbus, Ohio, US; abstract no. 16852k, 'GINSENG SAPONINS AS STIMULATORS OF HAIR GROWTH'
- CHEMICAL ABSTRACTS, vol. 115, no. 22, 2 Décembre 1991, Columbus, Ohio, US; abstract no. 239325, T. MINABE ET AL. 'HAIR GROWTH-STIMULATING PREPARATIONS CONTAINING CAMP, PYRUVIC ACID, FERMENTATION METABOLITES, AND NATURAL PRODUCTS'
- CHEMICAL ABSTRACTS, vol. 115, no. 22, 2 Décembre 1991, Columbus, Ohio, US; abstract no. 239293c, H. TANAKA ET AL. 'EFFECT OF PANAX GINSENG ON THE PRODUCTION OF GLYCOSAMINOGLYCANS IN CULTURED HUMAN SKIN FIBROBLAST'
- CHEMICAL ABSTRACTS, vol. 113, no. 16, 15 Octobre 1990, Columbus, Ohio, US; abstract no. 138316r, K. KUMAZAWA 'HAIR GROWTH STIMULATING PREPARATIONS CONTAINING LYSOZYME CHLORIDE'
- PATENT ABSTRACTS OF JAPAN vol. 11, no. 300 (C-449)29 Septembre 1987 & JP,A,62 093 217 (SHISEIDO CO LTD) 18 Octobre 1985
- CHEM. PHARM. BULL. vol. 31, no. 9 , 18 Février 1983 pages 3205 - 3209 TOSHINOBU MORITA ET AL. 'CHEMICAL AND MORPHOLOGICAL STUDY ON CHINESE PANAX JAPONICUS C. A. MEYER' cité dans la demande

## Description

La présente invention concerne une nouvelle composition cosmétique ou dermatologique contenant au moins une saponine de type ginsenoside et ses applications notamment pour le soin des cheveux.

Les saponines, notamment celles issues des plantes du type Panax, ont fait l'objet de nombreuses études. L'article paru dans Chem. Pharm. Bull. 31, 9, 3205-3209 (1983) présente une étude chimique et morphologique des saponines tirées des rhizomes de panax japonicus récolté en Chine et les compare à celles tirées des espèces japonaises. Il met notamment en évidence la différence importante de composition en saponines selon les espèces.

Parmi les études consacrées aux saponines notamment de type ginsenosides, on citera tout particulièrement une étude récente parue dans Journal of Ethnopharmacology, 36, (1992), 27-38, faisant la synthèse de recherches effectuées en chimie sur le ginseng. Cet article cite, en particulier, diverses activités pharmacologiques des ginsengs, notamment celles du Panax notoginseng ou San-chi (produit I des tableaux 1 et 2), ainsi que les paramètres physicochimiques des différents ginsenosides (cf. tableau 4).

La demanderesse a fait une étude comparée de l'effet de différentes saponines et montré, en particulier, l'intérêt des saponines de San-chi comparées à celles du ginseng.

Cette étude a conduit la demanderesse à découvrir que de nouvelles compositions cosmétiques ou pharmaceutiques contenant une saponine de type ginsenoside dénommée G-Rb₁ et répondant à la structure suivante 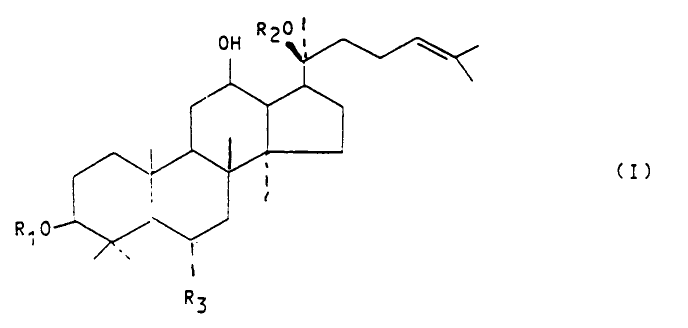 dans laquelle
R₁ = Glc(2-1)Glc,
R₂ = Glc(6-1)Glc,
R₃ = H
où Glc désigne un groupement β-D glucopyranosyle,
présentent une activité particulièrement intéressante pour freiner et/ou retarder la chute des cheveux. Elle a également découvert que cette saponine pouvait avantageusement être combinée à d'autres saponines, notamment aux saponines présentes dans panax notoginseng ou San-chi.

Elle a, en outre, découvert que des effets particulièrement avantageux en ce qui concerne la limitation de la chute des cheveux et/ou la repousse des cheveux étaient obtenus lorsque la saponine G-Rb₁, en combinaison ou non avec les autres saponines citées ci-dessus, était associée dans la composition cosmétique ou pharmaceutique à de la cépharantine.ou à de l'oxyacanthine.

Selon un premier aspect, la présente invention couvre une composition cosmétique ou dermatologique, en particulier destinée à favoriser la pousse des cheveux, caractérisée en ce qu'elle contient à titre d'ingrédient actif un extrait de plante Panax notoginseng ou Sanchi contenant au moins une saponine de formule (I): 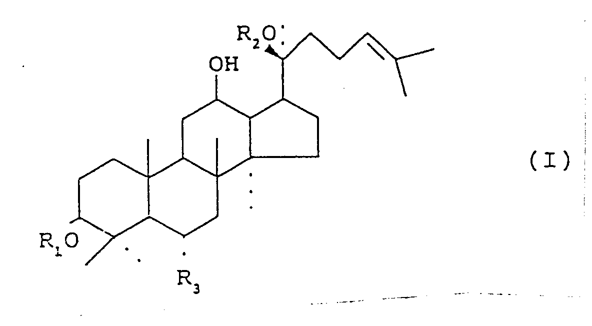 où
R₁ = -Glc(2-1)Glc ; R₂ = -Glc(6-1)Glc ; R₃ = H ; la saponine étant dénommée G-Rb₁,
Glc désignant un groupement β-D-glucopyranosyle,
la teneur de l'extrait végétal en saponine G-Rb₁ étant comprise entre 2 et 60 %, de préférence de 10 à 60 % et de préférence encore de 20 à 60 %, en poids par rapport au poids dudit extrait et en ce que la concentration totale en saponines précitées ou en extrait végétal précité est comprise entre 0,001 % et 2 % en poids par rapport au poids total de la composition.

Selon une variante de réalisation, l'extrait végétal contient en outre au moins une autre saponine répondant à la formule (I) précitée, où :
R₁ = -Glc(2-1)Glc ; R₂ = -Glc ; R₃ = H ; la saponine étant dénommée G-Rd, ou
R₁ = -Glc ; R₂ = -Glc(6-1)Glc ; R₃ = H ; la saponine étant dénommée Gy-XVII, ou
R₁ = -Glc(2-1)Glc ; R₂ = -Glc(6-1)Glc(6-1)Xyl ; R₃ = H ; la saponine étant dénommée N-R₄, ou
R₁ = H ; R₂ = -Glc ; R₃ = -O-Glc(2-1)Rha, la saponine étant dénommée G-Re, ou
R₁ = H ; R₂ = -Glc ; R₃ = -O-Glc, la saponine étant dénommée G-Rg₁, ou
R₁ = H ; R₂ = H ; R₃ = -O-Glc ; la saponine étant dénommée G-Rh₁, ou
R₁ = H ; R₂ = -Glc ; R₃ = -O-Glc(2-1)Xyl, la saponine étant dénommée N-R₁,
Glc, Xyl et Rha désignant respectivement un groupement β-D-glucopyranosyle, β-D-xylopyranosyle et α-L-rhamnopyranosyle.

Selon une autre variante de réalisation, l'extrait végétal contient :
de 2 % à 60 % en poids de saponine G-Rb₁
de 2 % à 60 % en poids de saponine G-Rg₁
de 0 % à 15 % en poids de saponine G-Rd
de 0 % à 15 % en poids de saponine N-R₁
de 1 % à 10 % en poids de saponine G-Re
par rapport au poids total de l'extrait.

Selon une variante de réalisation, l'extrait végétal contient :
de 10 % de 60 % en poids de saponine G-Rb₁
de 10% à 60 % en poids de saponine G-Rg₁
de 0 % à 15 % en poids de saponine G-Rd
de 0 % à 15 % en poids de saponine N-R₁
de 1 % à 10 % en poids de saponine G-Re
par rapport au poids total de l'extrait.

Dans les compositions précitées, l'extrait végétal est un extrait de Panax notoginseng ou San-chi, de préférence un extrait de racines ou de cormus.

Selon encore une autre variante de réalisation, la composition est caractérisée en ce que la concentration totale en extrait végétal précité est comprise entre 0,001 % et 2 % en poids par rapport au poids total de la composition.

Selon une autre variante, la concentration en extrait végétal précité est comprise entre 0,1 et 2 % en poids par rapport au poids total de la composition.

Selon encore d'autres variantes de réalisation particulièrement avantageuses, les compositions contiennent en outre de la cépharanthine ou de l'oxyacanthine ou l'un de leurs dérivés ou un extrait de plante en contenant, tel qu'un extrait de Stephania cepharantha ou de Berberis.

En outre, les différentes variantes de réalisation particulières exposées ci-après sont applicables aux différents aspects de l'invention. Ainsi, selon l'une de ces variantes, les compositions contiennent de 0,001 à 2 % en poids de cépharanthine ou d'oxyacanthine ou de l'un de leurs dérivés, ou d'un extrait de plante en contenant.

Selon une autre variante de réalisation particulière, la concentration en cépharanthine ou en oxyacanthine ou en l'un de leurs dérivés dans la composition est sensiblement égale ou inférieure, en particulier jusqu'à 10 fois inférieure à celle de la ou des saponines précitées.

Selon une variante de réalisation, la composition cosmétique ou dermatologique selon l'invention, comprend en outre au moins une autre substance active, à une concentration efficace, choisie parmi la quinine ou ses dérivés, les rubéfiants tels que le nicotinate de méthyle, un surnageant de culture de fibroblastes de papilles, tel que défini dans le document EP-A-272 920, des hydrolysats de kératine, des oligo-éléments tels que zinc, sélénium, cuivre, des inhibiteurs de 5-α-réductase tels que : progestérone, cyprotérone acétate, acide azélaïque et ses dérivés, un 4-méthyl-4-azastéroïde, en particulier la 17-β-N,N-diéthylcarbamoyl-4-méthyl-4-aza-5-α-androstan-3-one, ou encore un extrait de Serenoa repens.

Selon une autre variante, les compositions de l'invention contiennent en outre de l'acide hyaluronique, de préférence à une concentration comprise entre 0,01 et 1 % en poids.

Selon un mode particulier de réalisation de l'invention, l'extrait végétal contenant les saponines de formule (I) précitées est obtenu selon le procédé décrit ci-après à titre indicatif, mais nullement limitatif. On extrait la matière sèche au moyen d'un solvant choisi parmi le groupe constitué par : l'eau, les alcools comportant de préférence de 1 à 4 atomes de carbone, les esters organiques comportant de préférence de 3 à 6 atomes de carbone ; ou d'un solvant mixte à base d'un mélange quelconque des solvants précités.

Avantageusement, le solvant d'extraction primaire est le méthanol, l'éthanol, un mélange méthanol-eau ou un mélange éthanol-eau.

Le rapport de la matière végétale à l'agent d'extraction n'est pas critique et généralement sera compris entre 1 : 5 et 1 : 20 parties en poids.

L'extraction primaire précitée s'effectue à des températures comprises entre la température ambiante et le point d'ébullition du solvant utilisé pour l'extraction.

De préférence, l'extraction primaire est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée puis concentrée et/ou évaporée à sec sous pression réduite. On obtient ainsi un premier extrait selon l'invention riche en saponines.

Suivant une variante particulière, l'utilisation selon l'invention est relative à un mélange de saponines précitées. On obtient en particulier un mélange de saponines selon l'invention à partir du premier extrait concentré ou sec précité en opérant comme indiqué ci-après. Le premier extrait précité est introduit puis agité dans un solvant apolaire de préférence miscible avec le solvant d'extraction primaire, tel qu'un éther ou une cétone de faible poids moléculaire, en particulier l'éther éthylique ou isopropylique, l'acétone ou la méthyl-éthyl-cétone. La quantité de solvant apolaire est, en poids, généralement de 5 à 100 parties pour une partie d'extrait primaire. L'insoluble et/ou le précipité formé contient principalement un mélange de saponines selon l'invention.

Avantageusement, le mélange de saponines obtenu précédemment est purifié par un procédé quelconque à la portée de l'homme de l'an.

En particulier l'insoluble et/ou le précipité précité est remis en solution dans 20 fois environ son poids d'eau. La solution aqueuse est ensuite extraite 3 à 4 fois par un alcool peu soluble dans l'eau, tel que le butanol saturé en eau, par exemple en proportion 1/1 en volume à chaque opération d'extraction. Les phases alcooliques sont réunies et évaporées sous pression réduite. Le résidu est dissous dans 10 fois environ son poids d'eau, la solution est ensuite dialysée contre de l'eau pure pendant 4 à 5 jours. Le contenu de la cellule de dialyse est lyophilisé. Eventuellement pour améliorer encore la purification du mélange de saponines obtenu, le lyophilisat est dissous dans du méthanol, puis jeté dans de l'éther éthylique. On recueille le précipité formé. Les saponines peuvent également être extraites de tissus végétaux en culture (culture in vitro de racines ou de cals).

Lorsque la composition cosmétique selon l'invention contient de la cépharantine, cette dernière est avantageusement obtenue par extraction à partir de plante du genre Stephania, en particulier Stephania tetrandra, Stephania cepharanta, Stephania epigeae, Stephania sinica, Stephanie Delayavii, Stephania sasakii hayata, Stephania erecta, en utilisant des méthodes décrites par exemple dans US-A-2 206 607 ; US-A-2 248 241.

Selon un autre aspect, l'invention couvre encore l'utilisation d'une composition telle que précédemment définie pour favoriser la pousse des cheveux et/ou retarder leur chute, notamment dans le traitement de l'alopécie androgénogénétique, pour le soin de la peau, en particulier pour restaurer, préserver et/ou renforcer la fonction protectrice de la peau, notamment la fonction de barrière hydrique, pour la régénération de l'épiderme ou pour la prévention ou le traitement des rides.

Les compositions cosmétique ou dermatologique, conformément à la présente invention peuvent être appliquées par voie topique pour l'activité énoncée ci-dessus, en particulier dans des compositions se présentant sous forme de crème, de gel ou de lotion destinées à l'application sur le cuir chevelu.

Selon un autre aspect, la présente invention fournit également un procédé de traitement destiné à favoriser la pousse des cheveux, à retarder leur chute, notamment dans le traitement de l'alopécie androgénogénétique, ou à combattre le prurit, notamment le prurit du cuir chevelu, pour le soin de la peau, en particulier pour restaurer, préserver et/ou renforcer la fonction protectrice de la peau, notamment la fonction de barrière hydrique, pour la régénération de l'épiderme ou pour la prévention ou le traitement des rides, caractérisé en ce qu'il comprend l'application sur la zone à traiter, d'une quantité efficace, pour réaliser ledit effet recherché, d'au moins une saponine de formule (I) précitée ou d'un extrait végétal en contenant, ladite saponine ou ledit extrait étant éventuellement associé(e) à de la cepharantine ou de l'oxyacanthine ou un de leurs dérivés ou à un extrait de plante en contenant, tel qu'un extrait de Stephania cepharantha ou de Berberis.

Selon un autre aspect, l'invention fournit encore un procédé de fabrication d'une composition cosmétique ou dermatologique, destinée en particulier à stimuler la pousse des cheveux, à retarder leur chute, notamment dans le traitement de l'alopécie androgénogénétique, ou à combattre le prurit, notamment le prurit du cuir chevelu, pour le soin de la peau, en particulier pour restaurer et préserver et/ou renforcer la fonction protectrice de la peau, notamment la fonction de barrière hydrique, pour la régénération de l'épiderme ou pour la prévention ou le traitement des rides, caractérisé en ce qu'il comprend l'emploi de saponine de formule (I) ou d'un extrait de plante en contenant, ladite saponine ou ledit extrait de plante étant éventuellement associé(e) à de la cepharantine ou de l'oxyacanthine ou un de leurs dérivés ou à un extrait de plante en contenant, tel qu'un extrait de Stephania cepharantha ou de Berberis, que l'on mélange dans un excipient, véhicule ou support dermatologiquement ou cosmétiquement acceptable.

Selon d'autres variantes de réalisation, on utilise les diverses variantes de réalisation qui ont été précédemment énoncées pour les autres aspects de l'invention.

Les pourcentages sont donnés en poids sauf indication contraire.Les exemples qui suivent sont donnés à titre illustratif et nullement limitatif de la présente invention.

### Exemple 1

### Mise en évidence de l'intérêt des saponines de San-chi et comparaison avec celles du Panax ginseng.

On a testé sur une lignée de kératinocytes humains transformés l'activité individuelle de saponines de San-chi, et d'un mélange de saponines extrait de San-chi, en comparaison avec une saponine G-Rc abondante dans le Panax ginseng.

Le protocole suivant a été suivi :
J = 1 : ensemencement 50 000 cellules/boîte dans du milieu EMEM + 1 % SVF,
J = 0 : changement de milieu + apport des produits à tester,
J = 6 : numération cellulaire.

Les résultats sont consignés dans le tableau 1 ci-dessous.

**TABLEAU 1**

| Produit µg/ml | | Activité | Statistique |
|---|---|---|---|
| G-Rb₁ | 25 | + 4,4 | NS |
| | 50 | + 5,6 | NS |
| | 100 | + 17,7 | S |
| G-Rd | 25 | - 2,4 | NS |
| | 50 | + 8,6 | NS |
| | 100 | - 25,6 | S |
| G-Re | 25 | + 1,5 | NS |
| | 50 | + 7,6 | NS |
| G-Re | 25 | + 1,5 | NS |
| | 50 | + 7,6 | NS |
| | 100 | + 9,7 | NS |
| G-Rg₁ | 25 | - 6 | NS |
| | 50 | - 4,9 | NS |
| | 100 | + 4,1 | NS |
| San-chi saponines | 25 | - 2,3 | NS |
| | 50 | + 3,8 | NS |
| | 100 | + 11,5 | S |
| G-Rc | 25 | + 16,3 | NS |
| | 50 | - 30,2 | S |
| | 100 | - 97,0 | S |

Il apparaît donc que le Rb₁ est responsable de l'activité promitotique.

Ces données deviennent particulièrement intéressantes et fondamentales lorsqu'on les rapproche de la composition en ginsenosides du San-chi comparé au White Ginseng.

Les valeurs données dans une publication très récente de Acta Botanica Yunnanica 1988, I, 47-62 sont les suivantes (en %) :

| Ginsenosides | San-chi | White Ginseng |
|---|---|---|
| G-Rb₁ | 1,8 | 0,47 |
| G-Rd | 0,2 | 0,15 |
| G-Re | 0,15 | 0,15 |
| G-Rg₁ | 1,9 | 0,21 |
| G-Rc | - | 0,26 |
| (le White Ginseng est une racine de Panax ginseng qui est simplement pelée et séchée). | | |

Ce tableau montre que, si le taux en Rd cytotoxique est voisin, celui du ginsenoside Rb₁ responsable de l'activité est à un taux 4 fois supérieur dans San-chi. Cela confirme l'intérêt du choix particulier du San-chi par rapport au Panax ginseng. De plus, le White Ginseng possède environ 0,6 de G-Rc qui est très cytotoxique alors que le San-Chi n'en contient pratiquement pas.

### Exemple 2

### Lotion dermatologique pour traiter l'alopécie androgénogénétique

On prépare une lotion (dénommée A) selon l'invention contenant :

| | |
|---|---|
| Alcool absolu | 32,00 g |
| Butylhydroxyanisole | 0,001 g |
| Cépharantine | 0,10 g |
| Saponines de panax notoginseng (San-chi) | 0,10 g |
| Eau sauvage® | 0,35 g |
| Ceraphyl 60® | 0,08 g |
| Cremophor Rh 40® | 0,40 g |
| Excipient aqueux q.s.p. | 100 g |

### Exemple 3

### Lotion capillaire tonique antichute selon l'invention

Cette lotion répond à la formulation suivante avec les proportions indiquées ci-dessous en poids :

| | |
|---|---|
| Cépharantine | 0,15 g |
| Ginsenoside Rb₁ | 0,20 g |
| BHA | 0,05 g |
| Ceraphyl 60® | 0,07 g |
| Crémophor RH₄O | 0,5 g |
| Ethanol | 35 g |
| Acide hyaluronique | 0,1 g |
| Excipient aqueux parfumé q.s.p. | 100 g |

### Exemple 4

### Préparation d'une lotion capillaire coiffante antichute selon l'invention.

On prépare une composition répondant à la formulation suivante avec les proportions ci-dessous données en poids :

| | |
|---|---|
| Cépharantine | 0,1 g |
| Saponines de panax notoginseng | 0,12 g |
| Propylèneglycol | 3,00 g |
| Cremophor RH₄O | 0,5 g |
| Panthénol | 0,1 g |
| Ethanol | 32 g |
| Excipient aqueux parfumé q.s.p. | 100 g |

### Exemple 5

### Préparation d'un gel traitant antichute antipelliculaire et antiprurit

On utilise un mélange dénommé ci-dessous S de saponines contenant les proportions suivantes en poids :

| | |
|---|---|
| G-Rb₁ | 2 |
| G-Rd | 0,2 |
| G-Re | 1 |
| G-Rg₁ | 2 |
| N-R₁ | 0,2 |

pour préparer le gel répondant à la formulation ci-dessous où les quantités sont exprimées en poids

| | |
|---|---|
| Cépharantine | 0,05 g |
| Mélange S | 0,02 g |
| Ethanol | 30 g |
| Cremophor RH₄O® | 0,5 g |
| Gel de Carbopol 980® à 2 % | 50 g |
| Excipient aqueux q.s.p. | 100 g |

Ce gel s'applique 3 fois par semaine, de préférence le soir pendant 15 min en massage. Rinçage à l'eau.

### Exemple 6

### Lotion tonique après rasage

| | |
|---|---|
| Extrait de Panax notoginseng | 0,05 g |
| Cépharanthine | 0,05 g |
| Alcool 90 v/v | 30,- g |
| Alpha-bisabolol | 0,1 g |
| Excipient aqueux parfumé q.s.p. | 100,- g |

### Exemple 7

### Emulsion anti-ride

| | |
|---|---|
| Ginsenoside G-Rb₁ | 0,6 g |
| Oxyacanthine | 0,1 g |
| Excipient émulsionnant q.s.p. | 100 g |

### Example 8

### Lait raffermissant pour le corps

| | |
|---|---|
| Ginsenoside G-Rb₁ | 0,1 g |
| Ginsenoside G-Rg₁ | 0,1 g |
| Excipient pour lait, q.s.p. | 100,- g |

### Exemple 9

### Shampooing pour stimuler la pousse des cheveux

| | |
|---|---|
| Saponine de Panax notoginseng | 0,3 g |
| Diéthanolamine de coprah | 2 g |
| Lauryléthersulfate de sodium | 0,5 g |
| Alkylglucoside | 15,- g |
| Parahydroxybenzoate de méthyle | 0,5 g |
| Excipient aqueux parfumé, q.s.p. | 100,- g |

### Exemple 10

### Etude clinique d'une composition selon l'invention

L'étude est réalisée sur la composition A de l'exemple 2. Les résultats sont comparés à ceux obtenus avec un placebo constitué d'une composition identique à A, mais ne contenant ni cépharantine ni extrait de San-chi.

### 1. Protocole des essais

### . Critères d'inclusions

- sujets volontaires de sexe masculin, en bonne santé avant une chute chronique et importante pris au hasard lors de la consultation du cuir chevelu.
- Ages extrêmes 21-44 ans pour la lotion, moyenne d'âge 33,3 ans
   22-45 ans pour le placebo, moyenne d'âge 34,2 ans

### . Posologie et administration

La lotion A et son placebo ont été appliqués 2 fois par jour à raison de 7 pulvérisations chaque fois, sans discontinuité, pendant 6 mois, sur le sommet du crâne surtout, suivies d'un léger massage de quelques minutes. Aucun traitement n'a été associé. Il y a eu 10 lotions et 10 placebos remis au hasard, sans connaissance de la nature des flacons par l'expert.

Tous les sujets étaient en parfaite santé.

Le stade de leur chute de cheveux s'échelonnait, selon la classification d'Hamilton, ainsi :

| | |
|---|---|
| . Stade II | = 1 cas |
| . Stade III | = 6 cas |
| . Stade III vertex | = 5 cas |
| . Stade IV | = 7 cas |
| . Type féminin stade I+ | = 1 cas |

### 2. Analyse des résultats

La chute des cheveux a été appréciée grâce à 3 paramètres :
. l'impression subjective du patient,
. le test de traction,
. le trichogramme.
   - L'impression subjective est sujette à caution car fonction de l'anxiété et l'obsession liées à la chute des cheveux.
   - Le test de traction consiste à faire glisser entre 2 doigts des mèches de cheveux en trois points du cuir chevelu : vertex, tempe, occiput et à tirer selon une force régulière. On dénombre le nombre de cheveux venant à la traction. Ceci permet de vérifier la réalité de la chute.
   - Le trichogramme permet de vérifier l'action du produit par détermination de la formule pilaire après prélèvements de cheveux au niveau vertex, tempe, occiput.
   - Les tests de traction ont été réalisés à 0,3 mois, 6 mois.
   - Les trichogrammes à 0 et 6 mois.
   - Les sujets ont eu un entretien et un examen médical à 0, 3 mois, 6 mois afin d'évaluer la gravité de l'alopécie et son évolution.

A côté des effets sur la chute proprement dite l'action de la lotion sur des signes d'accompagnement a été évaluée.

Il s'agit :
- de l'état squameux ou pellicules,
- du prurit.

L'étude en double aveugle réalisée sur 20 sujets fait ressortir après 6 mois de traitement une action certaine de la lotion sur la chute des cheveux chez l'homme.

On peut noter également :
- un effet remarquable sur l'état squameux et le prurit,
- une application agréable.

## Revendications

1. Composition cosmétique ou dermatologique, en particulier destinée à favoriser la pousse des cheveux, caractérisée en ce qu'elle contient à titre d'ingrédient actif un extrait de plante Panax notoginseng ou Sanchi contenant au moins une saponine de formule (I) : 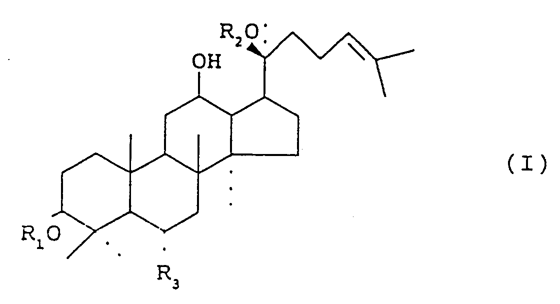 où
R₁ = -Glc(2-1)Glc ; R₂ = -Glc(6-1)Glc ; R₃ = H ; la saponine étant dénommée G-Rb₁,
Glc désignant un groupement β-D-glucopyranosyle,
la teneur de l'extrait végétal en saponine G-Rb₁ étant comprise entre 2 et 60 %, de préférence de 10 à 60 % et de préférence encore de 20 à 60 %, en poids par rapport au poids dudit extrait et en ce que la concentration totale en saponines précitées ou en extrait végétal précité est comprise entre 0,001 % et 2 % en poids par rapport au poids total de la composition.

2. Composition selon la revendication 1, caractérisée en ce que ledit extrait contient en outre au moins une autre saponine répondant à la formule (I) précitée où :
R₁ = -Glc(2-1)Glc ; R₂ = -Glc ; R₃ = H ; la saponine étant dénommée G-Rd, ou
R₁ = -Glc ; R₂ = -Glc(6-1)Glc ; R₃ = H ; la saponine étant dénommée Gy-XVII, ou
R₁ = -Glc(2-1)Glc ; R₂ = -Glc(6-1)Glc(6-1)Xyl ; R₃ = H ; la saponine étant dénommée N-R₄, ou
R₁ = H ; R₂ = -Glc ; R₃ = -O-Glc(2-1)Rha ; la saponine étant dénommée G-Re, ou
R₁ = H ; R₂ = -Glc ; R₃ = -O-Glc ; la saponine étant dénommée G-Rg₁, ou
R₁ = H ; R₂ = H ; R₃ = -O-Glc ; la saponine étant dénommée G-Rh₁, ou
R₁ = H ; R₂ = -Glc ; R₃ = -O-Glc(2-1)Xyl ; la saponine étant dénommée N-R₁,
Glc, Xyl et Rha désignant respectivement un groupement β-D-glucopyranosyle, β-D-xylopyranosyle et α-L-rhamnopyranosyle.

3. Composition selon l'une des revendications 1 à 2, caractérisée en ce ledit extrait contient :
de 2 à 60 % en poids de saponine G-Rb₁
de 2 à 60 % en poids de saponine G-Rg₁
de 0 à 15 % en poids de saponine G-Rd
de 0 à 15 % en poids de saponine N-R₁
de 1 à 10 % en poids de saponine G-Re
par rapport au poids total de l'extrait.

4. Composition selon l'une des revendications 1 à 3, caractérisée en ce ledit extrait contient :
de 10 % à 60 % en poids de saponine G-Rb₁
de 10 % à 60 % en poids de saponine G-Rg₁
de 0 % à 15 % en poids de saponine G-Rd
de 0 % à 15 % en poids de saponine N-R₁
de 1 % à 10 % en poids de saponine G-Re
par rapport au poids total de saponines de l'extrait.

5. Composition selon l'une des revendications 1 à 4, caractérisée en ce que ledit extrait est un extrait de racines ou de cormus.

6. Composition selon l'une des revendications 1 à 5, caractérisée en ce que la concentration en extrait précité est comprise entre 0,1 et 2 % en poids par raport au poids total de la composition.

7. Composition selon l'une des revendications 1 à 6, caractérisée en ce qu'elle contient en outre de la cépharanthine ou de l'oxyacanthine ou l'un de leurs dérivés ou un extrait de plante en contenant, tel qu'un extrait de Stephania cepharantha ou de Berberis.

8. Composition selon la revendication 7, caractérisée en ce qu'elle contient de 0,001 à 2 % en poids de cépharanthine ou d'oxyacanthine ou de l'un de leurs dérivés, ou d'un extrait de plante en contenant.

9. Composition selon l'une des revendications 7 ou 8, caractérisée en ce que la concentration en cépharanthine ou en oxyacanthine ou en l'un de leurs dérivés dans la composition est sensiblement égale ou inférieure, en particulier jusqu'à 10 fois inférieure à celle de la ou des saponines précitées.

10. Composition selon l'une des revendications 1 à 9, caractérisée en ce qu'elle comprend en outre au moins une substance active, à une concentration efficace, choisie parmi la quinine ou ses dérivés, les rubéfiants tels que le nicotinate de méthyle, un surnageant de culture de fibroblastes de papilles, des hydrolysats de kératine, des oligo-éléments tels que zinc, sélénium, cuivre, des inhibiteurs de 5-α-réductase tels que : progestérone, cyprotérone acétate, acide azélaïque et ses dérivés, un 4-méthyl-4-azastéroïde, en particulier la 17-β-N,N-diéthylcarbamoyl-4-méthyl-4-aza-5-α-androstan-3-one, ou encore un extrait de Serenoa repens.

11. Composition selon l'une des revendications 1 à 10, caractérisée en ce qu'elle contient en outre de 0,01 % à 1 % d'acide hyaluronique en poids.

12. Utilisation d'une composition selon l'une des revendications 1 à 11 comme agent cosmétique destiné à favoriser la pousse des cheveux et/ou retarder leur chute ou à soigner la peau, en particulier pour restaurer, préserver et/ou renforcer la fonction protectrice de la peau, notamment la fonction de barrière hydrique, à régénérer l'épiderme ou à prévenir ou traiter les rides.

13. Procédé de traitement cosmétique destiné à favoriser la pousse des cheveux et/ou à retarder leur chute, à soigner la peau, en particulier pour restaurer, préserver et/ou renforcer la fonction protectrice de la peau, notamment la fonction de barrière hydrique, à régénérer l'épiderme ou à prévenir ou traiter les rides, caractérisé en ce qu'il comprend l'application sur la zone à traiter, d'une quantité cosmétiquement efficace, pour réaliser ledit effet recherché, d'une composition selon l'une des revendications 1 à 11.

14. Utilisation d'un extrait de Panax notoginseng ou Sanchi pour la fabrication d'une composition pharmaceutique, notamment dermatologique, destinée au traitement de l'alopécie androgénogénétique, à combattre le prurit, notamment le prurit du cuir chevelu ou à prévenir ou traiter les rides.

## Claims

1. A cosmetic or dermatological composition intended in particular for promoting hair growth, characterized in that it contains, as active ingredient, a Panax notoginseng plant extract or Sanchi plant extract containing at least one saponin of formula (I): 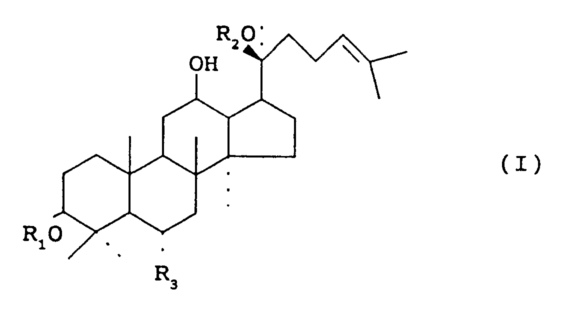 in which:
R₁ = -Glc(2-1)Glc, R₂ = -Glc(6-1)Glc and R₃ = H, the saponin being called G-Rb₁,
Glc denoting a β-D-glucopyranosyl group,
the proportion of saponin G-Rb₁ in the plant extract being between 2 and 60%, preferably from 10 to 60% and particularly preferably from 20 to 60% by weight, based on the weight of said extract, and in that the total concentration of abovementioned saponins or abovementioned plant extract is between 0.001% and 2% by weight, based on the total weight of the composition.

2. The composition according to claim 1, characterized in that said extract also contains at least one other saponin of formula (I) given above in which:
R₁ = -Glc(2-1)Glc, R₂ = -Glc and R₃ = H, the saponin being called G-Rd, or
R₁ = -Glc, R₂ = -Glc(6-1)Glc and R₃ = H, the saponin being called Gy-XVII, or
R₁ = -Glc(2-1)Glc, R₂ = -Glc(6-1)Glc(6-1)Xyl and R₃ = H, the saponin being called N-R₄, or
R₁ = H, R₂ = -Glc and R₃ = -O-Glc(2-1)Rha, the saponin being called G-Re, or
R₁ = H, R₂ = -Glc and R₃ = -O-Glc, the saponin being called G-Rg₁, or
R₁ = H, R₂ = H and R₃ = -O-Glc, the saponin being called G-Rh₁, or
R₁ = H, R₂ = -Glc and R₃ = -O-Glc(2-1)Xyl, the saponin being called N-R₁,
Glc, Xyl and Rha respectively being a β-D-glucopyranosyl, β-D-xylopyranosyl and α-L-rhamnopyranosyl group.

3. The composition according to one of claims 1 to 2, characterized in that said extract contains:
from 2 to 60% by weight of saponin G-Rb₁
from 2 to 60% by weight of saponin G-Rg₁
from 0 to 15% by weight of saponin G-Rd
from 0 to 15% by weight of saponin N-R₁
from 1 to 10% by weight of saponin G-Re
based on the total weight of the extract.

4. The composition according to one of claims 1 to 3, characterized in that said extract contains:
from 10% to 60% by weight of saponin G-Rb₁
from 10% to 60% by weight of saponin G-Rg₁
from 0% to 15% by weight of saponin G-Rd
from 0% to 15% by weight of saponin N-R₁
from 1% to 10% by weight of saponin G-Re
based on the total weight of saponins of the extract.

5. The composition according to one of claims 1 to 4, characterized in that said extract is an extract of roots or corms.

6. The composition according to one of claims 1 to 5, characterized in that the concentration of abovementioned extract is between 0.1 and 2% by weight, based on the total weight of the composition.

7. The composition according to one of claims 1 to 6, characterized in that it also contains cepharanthine or oxyacanthine, or a derivative thereof, or a plant extract in which it is present, such as an extract of Stephania cepharantha or Berberis.

8. The composition according to claim 7, characterized in that it contains from 0.001 to 2% by weight of cepharanthine or oxyacanthine, or a derivative thereof, or a plant extract in which it is present.

9. The composition according to one of claims 7 or 8, characterized in that the concentration of cepharanthine or oxyacanthine, or a derivative thereof, in the composition is approximately equal to or less than that of the abovementioned saponin or saponins, in particular up to 10 times less than that of the precited saponin or saponins.

10. The composition according to one of claims 1 to 9, characterized in that it also comprises an effective concentration of at least one active substance selected from quinine or derivatives thereof, rubefacients such as methyl nicotinate, a papilla fibroblast culture supernatant, keratin hydrolyzates, trace elements such as zinc, selenium and copper, 5-α-reductase inhibitors such as progesterone and cyproterone acetate, azelaic acid and derivatives thereof, a 4-methyl-4-azasteroid, in particular 17-β-N,N-diethylcarbamoyl-4-methyl-4-aza-5-α-androstan-3-one, or else an extract of Serenoa repens.

11. The composition according to one of claims 1 to 10, characterized in that it also contains from 0.01% to 1% by weight of hyaluronic acid.

12. Use of a composition according to one of claims 1 to 11, as a cosmetic agent intended for promoting hair growth and/or slowing down hair loss or for treating the skin, in particular for restoring, preserving and/or strengthening the protective function of the skin, especially the water barrier function, for regenerating the epidermis or for preventing or treating wrinkles.

13. A method of cosmetic treatment intended for promoting hair growth and/or slowing down hair loss, for treating the skin, in particular for restoring, preserving and/or strengthening the protective function of the skin, especially the water barrier function, for regenerating the epidermis or for preventing or treating wrinkles, characterized in that it comprises applying, to the area to be treated, a composition according to one of claims 1 to 11 in a cosmetically effective amount for achieving said desired effect.

14. Use of an extract of Panax notoginseng or Sanchi for manufacturing a pharmaceutical composition, in particular a dermatological composition, intended for the treatment of androgenogenetic alopecia, or combating pruritus, especially pruritus of the scalp, or preventing or treating wrinkles.

## Patentansprüche

1. Kosmetische oder dermatologische Zusammensetzung, insbesondere zur Förderung des Haarwuchses, dadurch gekennzeichnet, daß sie als aktiven Bestandteil einen Extrakt der Pflanze Panax notoginseng oder Sanchi enthält, enthaltend zumindest ein Saponin der Formel (I): 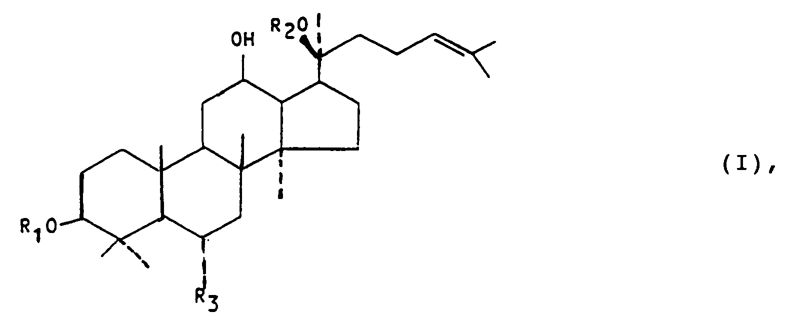 worin R₁ = -Glc(2-1)Glc; R₂ = -Glc(6-1)Glc; R₃ = H; wobei das Saponin als G-Rb₁ bezeichnet wird, Glc eine β-D-Glucopyranosyl-Gruppe darstellt, der Gehalt des Pflanzen-Extrakts an Saponin G-Rb₁ zwischen 2 und 60 %, vorzugsweise 10 bis 60 % und auch vorzugsweise 20 bis 60 %, bezogen auf die Masse des Extrakts, beträgt; und daß die Gesamtkonzentration der Saponine oder des Pflanzen-Extrakts zwischen 0,001 Masse-% und 2 Masse-%, bezogen auf die Gesamtmasse der Zusammensetzung, beträgt.

2. Zusammensetzung nach Anspruch 1, dadurch gekennzeichnet, daß der Extrakt außerdem zumindest ein anderes Saponin der Formel (I) enthält, worin R₁ = -Glc(2-1)Glc; R₂ = -Glc; R₃ = H; wobei das Saponin als G-Rd bezeichnet wird; oder R₁ = -Glc; R₂ = -Glc(6-1)Glc; R₃ = H; wobei das Saponin als Gy-XVII bezeichnet wird; oder R₁ = -Glc(2-1)Glc; R₂ = -Glc(6-1)Glc(6-1)Xyl; R₃ = H; wobei das Saponin als N-R₄ bezeichnet wird; oder R₁ = H; R₂ = -Glc; R₃ = -O-Glc(2-1)Rha; wobei das Saponin als G-Re bezeichnet wird; oder R₁ = H; R₂ = -Glc; R₃ = -O-Glc; wobei das Saponin als G-Rg₁ bezeichnet wird; oder R₁ = H; R₂ = H; R₃ = -O-Glc; wobei das Saponin als G-Rh₁ bezeichnet wird; oder R₁ = H; R₂ = -Glc; R₃ = -O-Glc(2-1)-Xyl; wobei das Saponin als N-R₁ bezeichnet wird; Glc, Xyl und Rha jeweils eine β-D-Glucopyranosyl-, β-D-Xylopyranosyl- und α-L-Rhamnopyranosyl-Gruppe bedeuten.

3. Zusammensetzung nach einem der Ansprüche 1 bis 2, dadurch gekennzeichnet, daß der Extrakt enthält:
2 bis 60 Masse-% Saponin G-Rb₁,
2 bis 60 Masse-% Saponin G-Rg₁,
0 bis 15 Masse-% Saponin G-Rd,
0 bis 15 Masse-% Saponin N-R₁,
1 bis 10 Masse-% Saponin G-Re,
bezogen auf die Gesamtmasse des Extrakts.

4. Zusammensetzung nach einem der Ansprüche 1 bis 3, dadurch gekennzeichnet, daß der Extrakt enthält:
10 bis 60 Masse-% Saponin G-Rb₁,
10 bis 60 Masse-% Saponin G-Rg₁,
0 bis 15 Masse-% Saponin G-Rd,
0 bis 15 Masse-% Saponin N-R₁,
1 bis 10 Masse-% Saponin G-Re,
bezogen auf die Gesamtmasse der Saponine des Extrakts.

5. Zusammensetzung nach einem der Ansprüche 1 bis 4, dadurch gekennzeichnet, daß der Extrakt ein Wurzel- oder Kormus-Extrakt ist.

6. Zusammensetzung nach einem der Ansprüche 1 bis 5, dadurch gekennzeichnet, daß die Konzentration des Extrakts zwischen 0,1 und 2 Masse-%, bezogen auf die Gesamtmasse der Zusammensetzung, beträgt.

7. Zusammensetzung nach einem der Ansprüche 1 bis 6, dadurch gekennzeichnet, daß sie außerdem Cepharanthin oder Oxyacanthin oder eines ihrer Derivate oder einen diese enthaltenden Pflanzen-Extrakt, wie einen Extrakt von Stephania cepharantha oder Berberis, enthält.

8. Zusammensetzung nach Anspruch 7, dadurch gekennzeichnet, daß sie 0,001 bis 2 Masse-% Cepharanthin oder Oxyacanthin oder eines ihrer Derivate oder eines diese enthaltenden Pflanzen-Extrakts enthält.

9. Zusammensetzung nach einem der Ansprüche 7 oder 8, dadurch gekennzeichnet, daß die Konzentration von Cepharanthin oder Oxyacanthin oder eines ihrer Derivate in der Zusammensetzung im wesentlichen kleiner, insbesondere bis zu 10-mal kleiner, oder gleich ist jener des oder der Saponine.

10. Zusammensetzung nach einem der Ansprüche 1 bis 9, dadurch gekennzeichnet, daß sie außerdem zumindest eine aktive Substanz in einer wirksamen Konzentration, ausgewählt aus Chinin oder seinen Derivaten, Rubefacientien, wie Methylnicotinat, einem Kulturüberstand von Papillenfibroblasten, Keratinhydrolysaten, Spurenelementen, wie Zink, Selen, Kupfer, 5-α-Reduktase-Inhibitoren, wie Progesteron, Cyproteronacetat, Azelainsäure und ihren Derivaten, einem 4-Methyl-4-azasteroid, insbesondere 17β-N,N-Diethylcarbamoyl-4-methyl-4-aza-5-α-androstan-3-on, oder auch einen Extrakt von Serenoa repens umfaßt.

11. Zusammensetzung nach einem der Ansprüche 1 bis 10, dadurch gekennzeichnet, daß sie außerdem 0,01 Masse-% bis 1 Masse-% Hyaluronsäure enthält.

12. Verwendung einer Zusammensetzung nach einem der Ansprüche 1 bis 11 als kosmetisches Mittel zur Förderung des Haarwuchses und/oder Verlangsamung von Haarausfall oder zur Hautpflege, insbesondere um die Schutzfunktion der Haut, spezifisch die wasserabstoßende Funktion, wiederherzustellen, aufrechtzuerhalten und/oder zu verstärken, zur Regeneration der Epidermis oder zur Verhinderung oder Behandlung von Falten.

13. Verfahren zur kosmetischen Behandlung zur Förderung des Haarwuchses und/oder zur Verlangsamung von Haarausfall, zur Hautpflege, insbesondere um die Schutzfunktion der Haut, spezifisch die wasserabstoßende Funktion, wiederherzustellen, aufrechtzuerhalten und/oder zu verstärken, zur Regeneration der Epidermis oder zur Verhinderung oder Behandlung von Falten, dadurch gekennzeichnet, daß es das Aufbringen einer kosmetisch zur Erzielung des gewünschten Effekts wirksamen Menge einer Zusammensetzung nach einem der Ansprüche 1 bis 11 auf die zu behandelnde Zone umfaßt.

14. Verwendung eines Extrakts von Panax notoginseng oder Sanchi bei der Herstellung einer pharmazeutischen, insbesondere dermatologischen Zusammensetzung zur Behandlung von Alopecia androgenogenetica, zur Bekämpfung von Pruritus, insbesondere Pruritus der Kopfhaut, oder zur Verhinderung oder Behandlung von Falten.
